# EUROPEAN PATENT SPECIFICATION

(11) **EP 1 542 748 B1**
(45) Date of publication and mention of the grant of the patent: **26.02.2014**
(21) Application number: 03796285.9
(22) Date of filing: 30.07.2003
(51) Int. Cl.: A61M 5/50, A61M 5/315

(54) **SINGLE-USE SYRINGE**
EINWEGSPRITZE
SERINGUE A USAGE UNIQUE

(30) Priority: 27.09.2002 US 256607
(43) Date of publication of application: 22.06.2005
(73) Proprietor: Becton Dickinson and Company, Franklin Lakes, New Jersey 07417-1880 (US)
(72) Inventor: CAPES, David, Francis, Singapore (SG); MENG, Steven, Lau, Choon, Singapore 576190 (SG)
(74) Representative: von Kreisler Selting Werner
(86) International application number: PCT/US2003/023756
(87) International publication number: WO 2004/028602

(56) References cited:
- WO-A-01/62319
- WO-A-02/070053
- US-A- 5 383 857
- US-A- 5 423 756
- US-A1- 2002 107 489
- US-B1- 6 217 550

## Description

### FIELD OF THE INVENTION

The present invention relates to a syringe having a breakable plunger rod to prevent further use of the syringe after fluid delivery or injection.

### BACKGROUND

Troughout the world the re-use of hypodermic syringe products which are intended for single-use only is instrumental in drug abuse and in the transfer of contagious diseases. Intravenous drug users who routinely share and re-use syringes are a high-risk group with respect to the AIDS virus. Also, the effects of multiple use are a major concern in some countries where the repeated use of syringe products during mass inoculation programs may be responsible for the spread of many diseases. Syringes are often recycled in developing countries without proper sterilization.

Many attempts have been made to remedy this problem. Some designs involve the inclusion of structure which will allow the destruction or defeating of the syringe function through a conscious act by the user, such as breaking a syringe or one of its components. In addition, there are single-use hypodermic syringes which become incapable of further use automatically upon delivery of the medication without any additional act on the part of the user.

A syringe corresponding to the first part of claim 1 is disclosed in US-A-5383857. This syringe has a barrel and a plunger comprising a plunger rod and a stopper. The plunger rod has a breakable connection between a proximal portion and a distal portion. The syringe barrel is contained in a moveable guard sheath that is maintained in a retracted position at the proximal end of the syringe barrel. The plunger rod has a release surface that extends laterally. When the plunger is advanced into the barrel, a clearance volume within the stopper will be reduced and latch arms of the guard sheath will be released from a flange of the syringe barrel by the release surface of the plunger.

WO 02/070053 A1 describes a syringe comprising a syringe barrel and a piston movable within the syringe barrel. The piston comprises a plunger rod having a proximal portion and a distal portion which are connected through breakable bridges designed to break if the forces exerted on the piston rod exceed a certain threshold. The syringe barrel is moveably comprised within a protective sleeve. An actuating ring having laterally extending wings is slidable on the protective sleeve.

### SUMMARY OF THE INVENTION

It is an object of the invention to provide a syringe having a plunger with a proximal portion and a distal portion connected by a breakable connection, wherein the proximal portion is prevented from impacting the distal portion after breaking the breakable connection, thereby avoiding squeezing out of fluid in the dead space of the syringe, which would result in spraying some of the fluid.

The syringe of the invention is defined by claim 1. Accordingly, it is characterized in that the projection on the proximal portion of the plunger rod is so positioned that after breaking of the breakable connection, the proximal portion of the plunger rod is prevented from impacting the distal portion, thereby avoiding squeezing out of fluid from a dead space between the plunger rod and the distal end of the barrel.

A syringe comprises a barrel having a fluid chamber, a proximal end, a distal end, and an elongate tip extending from the distal end having a passageway therethrough in fluid communication with the chamber, and a plunger rod. The plunger rod has a longitudinal axis, a proximal portion and a distal portion connected by a breakable connection. One of the proximal portion and the distal portion includes an axial projection having at least one transverse protuberance projecting therefrom. The protuberance is connected to the other of the proximal portion and the distal portion. The breakable connection is on the protuberance. The distal portion includes a stopper slidably positioned in fluid-tight engagement with an inside surface of the chamber for drawing fluid into and out of the chamber by movement of the plunger rod relative to the barrel. The breakable connection is strong enough to hold the proximal portion and the distal portion together during normal use of the syringe and breakable upon application of an additional force applied to the proximal portion along the longitudinal axis. Structure is provided for preventing the proximal portion from applying a distally directed force to the distal portion after the breakable connection is broken. This structure may include a projection on the proximal portion of the plunger rod configured to contact the barrel after the breakable connection is broken. The plunger rod may have a plurality of outwardly projecting ribs wherein one or all of the ribs contains a projection to limit the distal motion of the proximal portion of the plunger rod. The projection may be integral or separately attached to the plunger or barrel. The projection may also take the form of a transverse flange on the plunger rod. The plunger rod may contain a distally directed extension on the distal portion shaped to fit within the passageway of the elongate tip of the syringe barrel for displacing fluid from the passageway when the plunger is positioned distally with respect to the barrel.

### BRIEF DESCRIPTION OF THE DRAWINGS

Fig. 1 is an exploded perspective view of the plunger rod and barrel of a syringe of the present invention;
Fig. 2 is an enlarged perspective view of the plunger rod of the syringe of the present invention having a distal portion and a proximal portion connected by a breakable connection;
Fig. 3 is a partial cross-sectional side elevation view of a syringe assembly using the plunger rod of Fig. 2;
Fig. 4 is a side elevational view of the syringe of Fig. 4 illustrated with a force being applied to break the connection between the proximal and distal portions of the plunger rod;
Fig. 5 is a side elevational view of the syringe of Fig. 4 illustrating the separated proximal and distal ends of the plunger rod;
Fig. 6 is a side elevational view of the plunger rod;
Fig. 7 is a top plan view of the plunger rod of Fig. 6;
Fig. 8 is a perspective view of an alternative embodiment of the plunger rod of the present invention;
Fig. 9 is an enlarged partial perspective view of the plunger rod of Fig. 8;
Fig. 10 is a partial cross-sectional view of the plunger rod of Fig. 8 in a syringe barrel;
Fig. 11 is a partial cross-sectional view of another alternative embodiment of the syringe of the present invention; and
Fig. 12 is another alternative embodiment of the plunger rod of the present invention.

### DETAILED DESCRIPTION

While this invention is satisfied by embodiments in many different forms, there is shown in the drawings and will herein be described in detail preferred embodiments of the invention with the understanding that the present disclosure is to be considered as exemplary of the principles of the invention and is not intended to limit the invention to the embodiments illustrated. The scope of the invention will be measured by the appended claims and their equivalents.

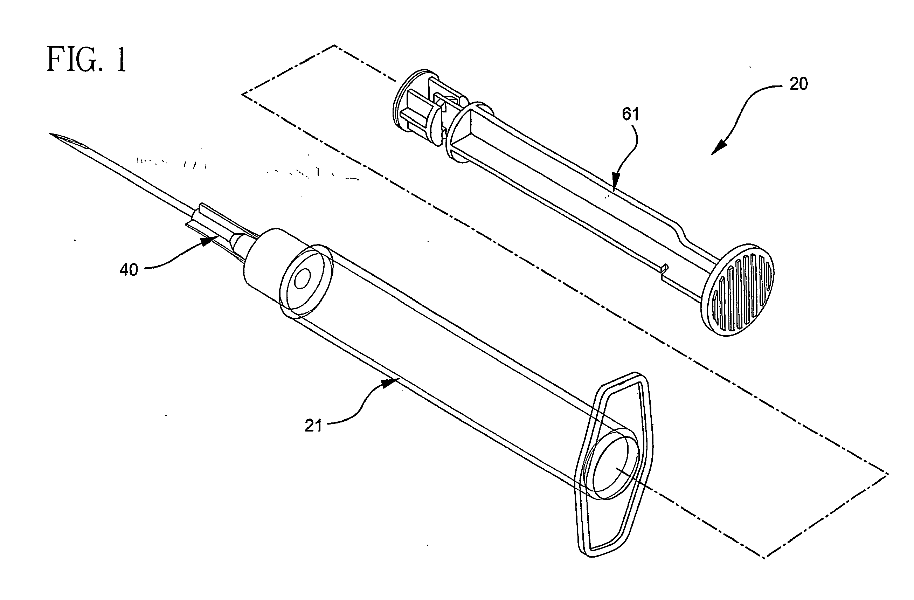

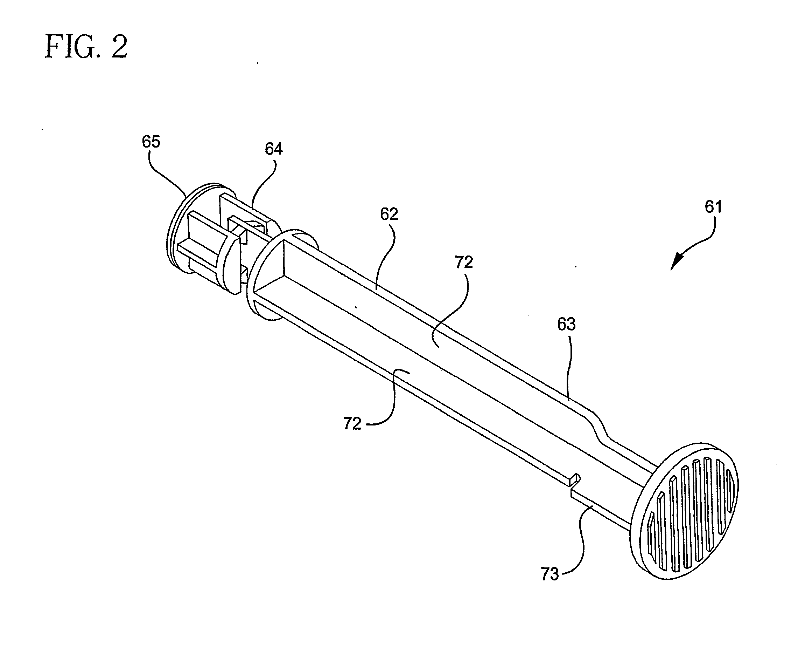

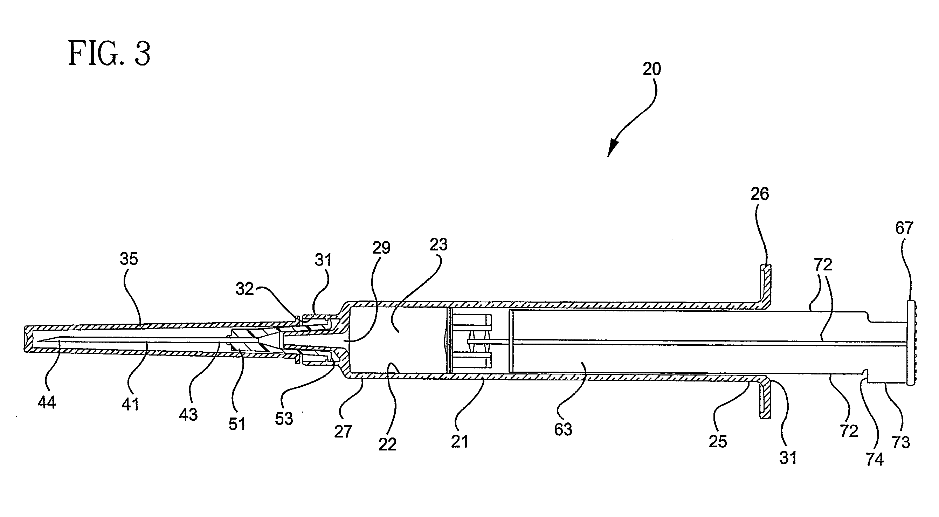

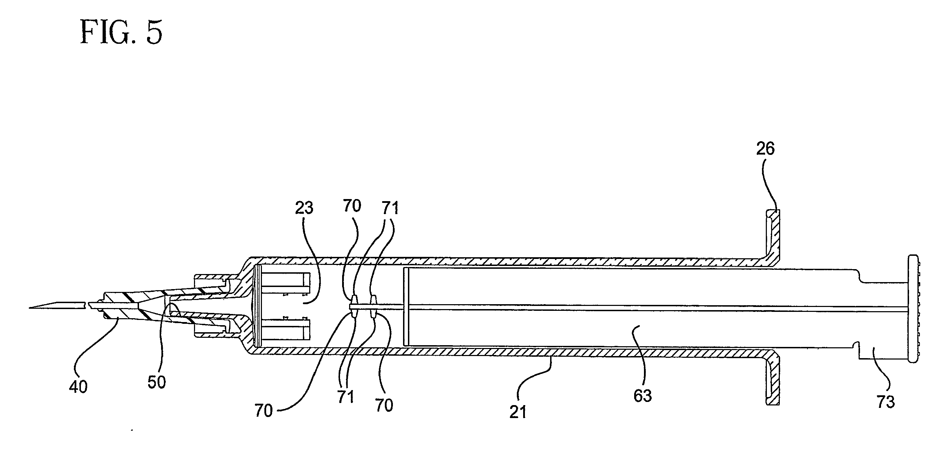

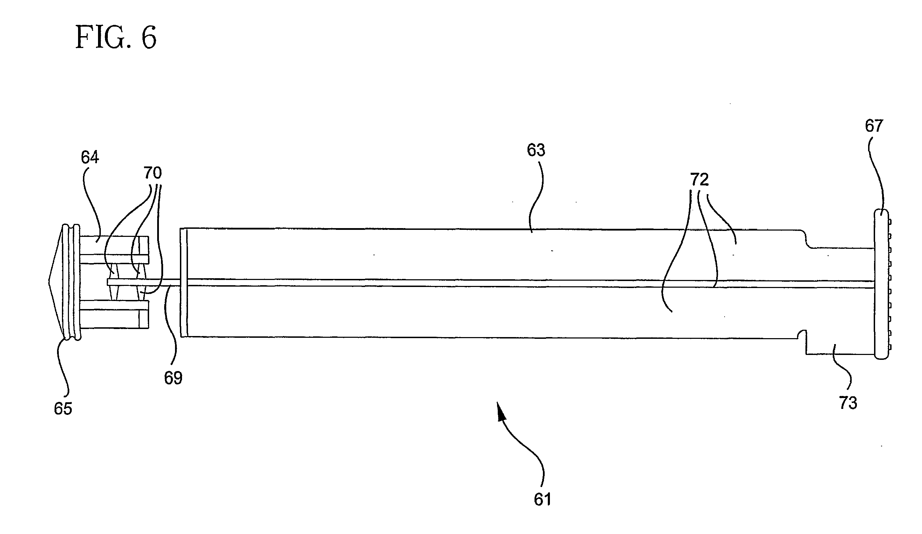

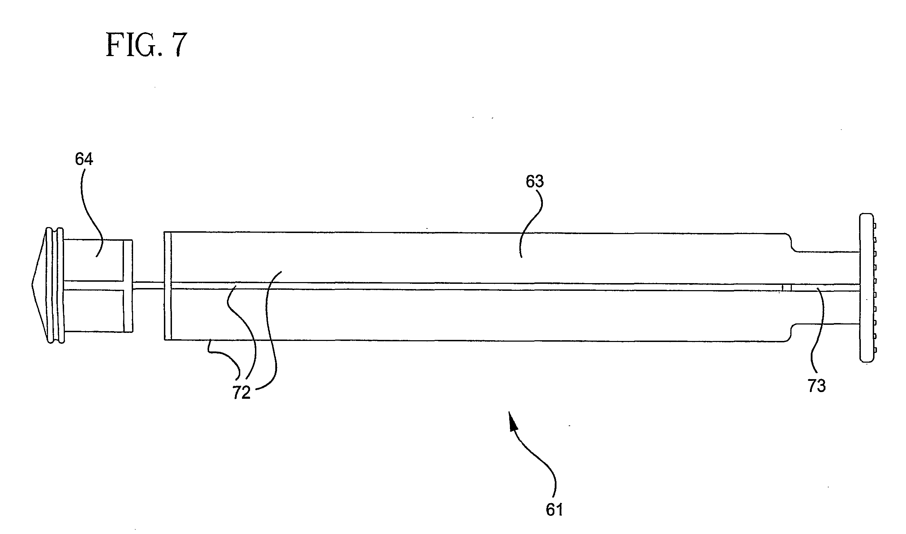

Adverting to Figs. 1-7 a syringe 20 includes a barrel 21 having a barrel inside surface 22, a fluid chamber 23, a proximal end 25, a distal end 27 and an elongate tip 28 extending from the distal end having a passageway 29 therethrough in fluid communication with the chamber. In this embodiment the elongate tip is preferably frusto-conically shaped.

Barrel 21 preferably includes a collar 31 having an inside surface 32 and at least one third to engage a needle hub.

The syringe of the present invention is intended to be used with a needle assembly 40 including a cannula 41 having a proximal end 43 a distal end 44 and a lumen 45 therethrough. The needle assembly also includes a hub 47 having an open proximal end 49 with a cavity 50 therein, and a distal end 51 joined to proximal end 43 of cannula 41 so that lumen 45 is in fluid communication with cavity 50. A removable needle shield 35 is provided to protect the cannula before use. It is within the purview of the present invention to include needle assemblies having one-piece construction wherein the cannula and the hub are formed of one piece.

Syringe barrel 22 preferably includes a collar 31 around the elongate tip having a thread on the inside surface of the collar. This configuration is often referred to as a locking luer collar. The needle assembly with the hub having outwardly extending projections 53 is placed on the distal end of the syringe by aligning the distal tip of the syringe with a cavity in the hub and moving the needle assembly toward the syringe so that the outward projections of the hub engage the thread. The needle assembly is then rotated or screwed into the locking luer collar so that the needle assembly is held tightly on the distal tip of the syringe barrel through interaction of the locking luer collar thread and the projections on the hub. This is an excellent structure for most applications since it allows for applying the appropriate sized needle assembly at the time of use and for changing needle assemblies during a procedure which may require two or more different sizes.

The syringe of the present embodiment includes a plunger rod 61 having an elongate body portion 62 defining longitudinal axis 66 and having a proximal portion 63, a distal portion 64 and a stopper 65 on the distal portion. The stopper is slidably positioned in fluid-tight engagement with the barrel inside surface of the chamber for drawing fluid in and out of the chamber by movement of the plunger rod relative to the barrel. The plunger rod extends outwardly from proximal end 25 of the barrel. The plunger rod is accessible outside of proximal end 25 of the barrel and is provided to move the stopper along the barrel to force fluid into and out of chamber 23 through passageway 29. Disc-shaped plunger rod flange 67 is provided as a convenient structure for applying forces to move the plunger rod with respect to barrel 21. A flange 26 is also provided at the proximal end of the barrel to facilitate handling and for maintaining the relative position of the barrel with respect to the plunger rod during fluid transfer using known procedures.

It is within the purview of the present invention to include plunger rods and stoppers which are separately formed or integrally formed of the same material or different materials such as in two-color molding, or separately formed of the same or different materials and joined together by mechanical means, adhesives, ultrasonic welding, heat sealing or other suitable means. Stoppers are preferably made of elastomeric material such as natural rubber, synthetic rubber, thermoplastic elastomers and combinations thereof. It is understood that the plunger of the present embodiment merely illustrates these many possibilities.

Proximal portion 63 and distal portion 64 of the plunger rod are connected by a breakable connection 68. Breakable connection 68 is strong enough to hold proximal portion 63 and distal portion 64 together during normal use of the syringe and is breakable upon application of additional force to the proximal portion.

In this embodiment, there are a one or more breakable connections connecting proximal portion 63 and distal portion 64. Specifically, proximal portion 63 includes a distal projection 69 having at least one transverse protuberance projecting therefrom. In this preferred embodiment, there are four transverse protuberances 70. The protuberances are connected to distal portion 64 and the breakable connection is on the protuberance. In this embodiment breakable connection 71 is preferably on the distal end of the protuberance. The distal projection may be circularly shaped and fit into a cylindrically shaped recess in the distal portion. With this structure a single protuberance extending up to 360 degrees may be used. The structure may also be reversed so that the projection extends proximally from the distal portion toward the proximal portion.

In this preferred embodiment, proximal portion 63 and distal portion 64 and the breakable connections are integrally molded of plastic material. A wide variety of plastic materials are suitable for molding the plunger with polystyrene, polypropylene and polyethylene being preferred. It is important to control the modulus of elasticity of material selected for the transverse protuberances which are part of the breakable connection between the proximal portion and the distal portion of the plunger rod. The breakable connection must break or fail before the proximal portion of the plunger contacts the distal portion of the plunger. If the modulus is too high the break will occur too easily, causing premature breakage. If the modulus is too low the breakable connection may not break because the proximal portion and the distal portion will contact each other to resist further relative movement between the proximal and distal portions. Also, careful controlling of the modulus will allow use of materials such as polypropylene which would not normally be used to form a breakable connection. It is preferred to have a modulus of elasticity be within the range of about 800MPa to 4000MPa.

The breakable connections can be anywhere along the protuberance, at its proximal end, its distal end, or in between, depending on, among other things, the geometry of the protuberance. A breakable connection can also be made by connecting the protuberance to the distal end or to the proximal end using a frangible adhesive. The protuberance may be very short and made entirely of adhesive or frangible material. The connection can also be made using a shear pin passing through the distal projection 69 and distal portion 64. The shear pin may be made of plastic with one or more notches or stress risers suitably placed to cause breaking at the desired force levels. The breakable connection between the proximal portion and the distal portion may also be accomplished by using a snap-fit arrangement a portion of which is damaged or broken when the desired force is applied. In this latter situation, the distal portion and the proximal portion can be individually molded and snapped together during the assembly process.

In use, the syringe of this embodiment can be filled from a vial, ampoule or other suitable container using known safe procedures. An important advantage of the present embodiment is that the plunger can be moved back and forth along the barrel as many times as necessary to properly fill the syringe barrel. For example, the syringe barrel may be filled with sterile water and then the sterile water can be injected into a vial containing a lyophilized medication which is then drawn back into the syringe barrel. Many single-use syringes in the prior art only allow one proximal motion of the plunger with respect to the barrel. With these single-use syringes, once the plunger is moved in a distal direction with respect to the barrel it can no longer be withdrawn. Therefore, mixing sterile water and a lyophilized medication as described above is not possible.

Another advantage of the present embodiment is that the plunger can be moved to its distal-most position with respect to the barrel and then moved proximally. Some prior art single use syringes automatically lock the plunger to the barrel when the plunger is moved to its distal-most position. These prior art designs can lead to unintentional locking of the plunger before use and can compromise filling and mixing procedures.

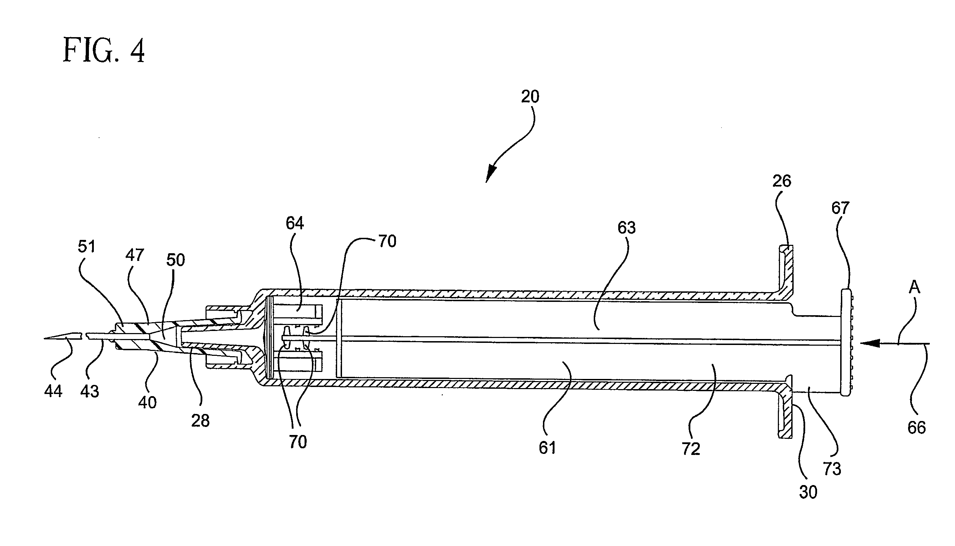

The liquid in the barrel can now be injected into a patient or delivered in another suitable manner such as through the pierceable septum of a catheter connector. Upon completion of the injection the user can apply an additional force indicated as A in Fig. 4, to the proximal portion. In this embodiment the breakable connections are broken by the application of a distally directed force A applied to the proximal end of the proximal portion along longitudinal axis 66. Force A is sufficient to break the breakable connections which causes the plunger rod to separate into two or more unusable pieces. In this enablement the proximal portion and the distal portion are separated as illustrated in Fig. 4.

After breaking the breakable connection the proximal portion of the plunger rod moves distally at high speed. If the proximal portion is permitted to impact the distal portion at this time, the distal portion may compress whatever fluid remains in the dead space between the plunger rod and the roof of the barrel. This action can cause some fluid in the dead space to be squeezed out of the passageway of the elongate tip at high speed, resulting in spraying some of the fluid from the nozzle. An important advantage of the present invention is that the syringe includes means for preventing proximal portion 63 of the plunger rod from applying distally directed force to distal portion 64 after the breakable connection is broken. Preferred means for preventing contact and thus the application of distally directed force by the proximal portion to the distal portion after the breakable connection is broken include providing a projection on the proximal portion configured to contact some portion of the barrel after the connection is broken and before contact with the distal portion can be made. In this preferred embodiment, the plunger rod 61 includes a plurality of outwardly projecting ribs 72 and a projection 73 on at least one of ribs 72. The projection is configured to contact some portion of the barrel after the breakable connection is broken and before the proximal portion can contact the distal portion of the plunger rod in order to prevent unwanted discharge of any residual contents of the syringe, chamber. According to the invention, projection 73 has a leading edge 74 which contacts proximal surface 30 on the flange 26 of the barrel. It is understood that any combination of projections and/or recesses on the plunger rod which it can interact with recesses and/or projections on the barrel to achieve the same result are within the purview of the present invention and that the structure illustrated in this preferred embodiment is merely representative of these many possibilities. In this embodiment, projection 73 is on one of ribs 72, however, a projection can be on one or more of the ribs provided.

An advantage of the present invention is that the breakable connection can be broken using any one-handed technique, for example, pressing on plunger rod flange 67 in direction A with the thumb of one hand while holding the syringe barrel and/or the syringe barrel flange with some or all of the remaining fingers. This is desirable over a two-handed technique wherein the barrel must be held by one hand and the plunger by another to carry out a breaking manipulation such as bending or twisting.

The embodiment of Figs. 1-7 is a significant advance over single-use syringes of the prior art. In particular, it allows multiple strokes of the plunger with respect to the barrel without automatically locking and rendering the syringe unusable. This design also allows the plunger to move to its distal-most position inside the barrel without automatically locking the plunger to the barrel. It also provides a mechanism to prevent or discourage re-use. The plunger is breakable so that its proximal and distal ends are separated and the syringe, can no longer be used to inject medication. Structure is provided to prevent unwanted force on the distal portion of the plunger rod after the breakable connection is broken in order to prevent unwanted discharge of residual medication from the chamber and/or passageway. Further, the plunger breaking feature is accomplished by a simple one-handed procedure.

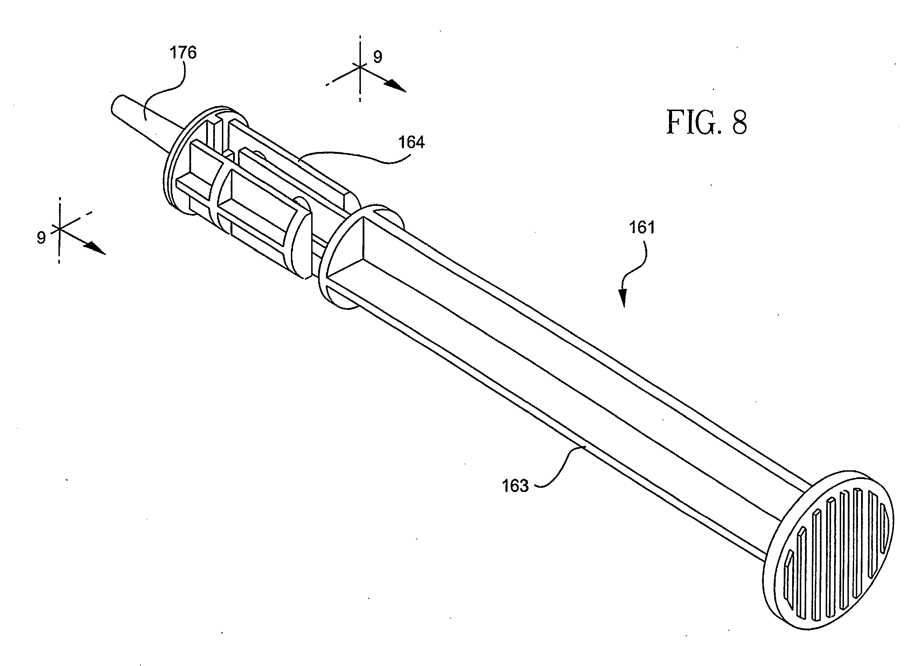

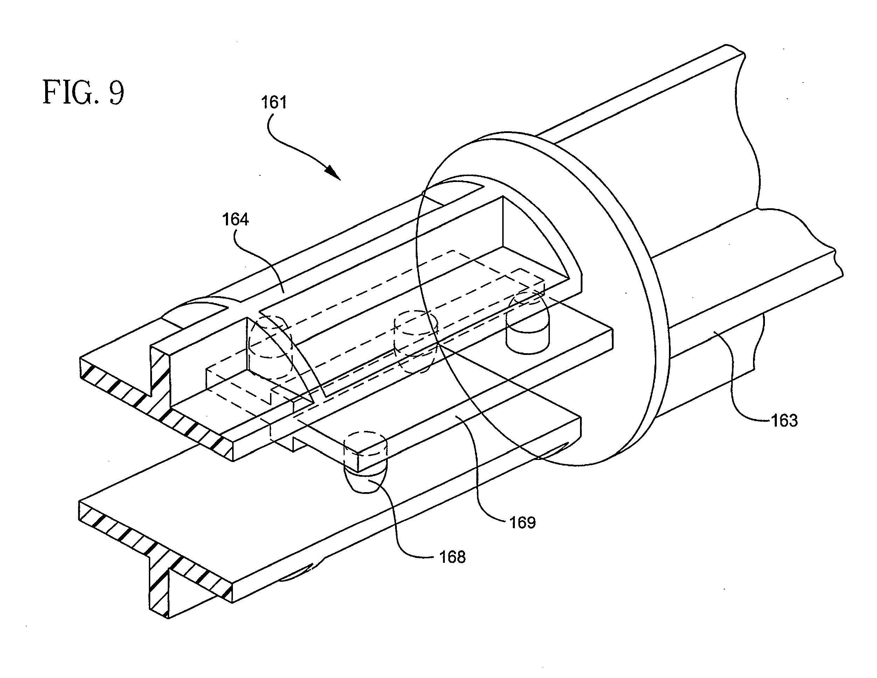

Figs. 8-10 illustrate an alternative embodiment of the present invention. In this embodiment, a syringe 120 comprising a barrel 121 having a fluid chamber 123, a proximal end 125, a distal end 127 and an elongate tip 128 extending from the distal end and having a passageway 129 therethrough in fluid communication with the chamber.

A plunger rod 161 having a longitudinal axis 166, a proximal portion 163 and a distal portion 164 connected by a breakable connection 168. It is preferred that either proximal portion 163 or distal portion 164 include an axial projection. In this embodiment an axial projection 169 is on proximal portion 163. Axial projection 169 includes at least one transverse protuberance projecting therefrom. In this embodiment, there are four transverse protuberances 170. The protuberances are connected to distal portion 164 and the breakable connection is on the protuberances. The transverse protuberances project from opposite sides of the axial projection. Also, in this embodiment, two of transverse protuberances 170 project in substantially the opposite direction from the other two transverse protuberances 170. It is preferable that proximal portion 163 and distal portion 164 and the transverse protuberances 170 which include breakable connections are integrally molded of plastic material.

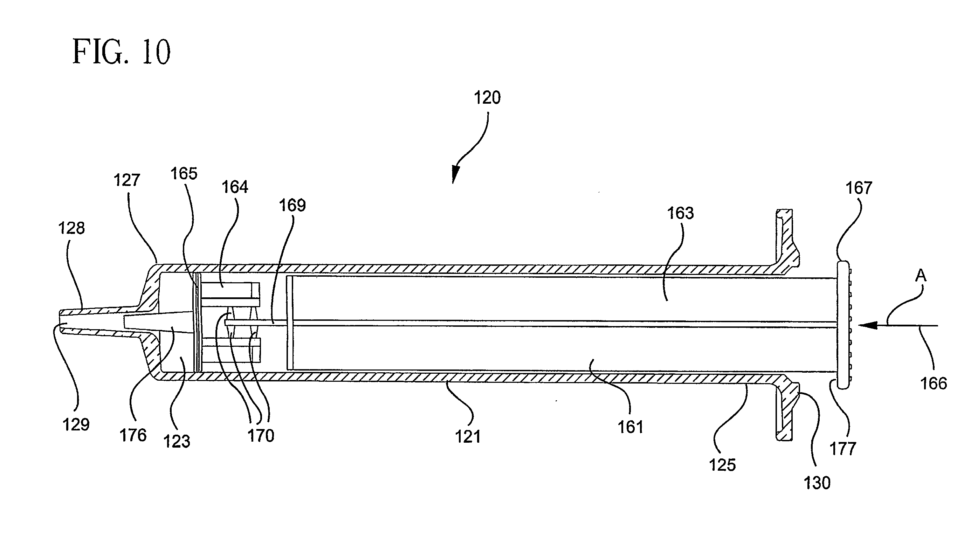

Plunger rod 161 in this embodiment functions similarly to plunger rod 61 in the embodiments of Figs. 1-7. Distal portion 164 includes a stopper 165 slidably positioned in fluid-tight engagement with an inside surface of chamber 123 for drawing fluid into and out of the chamber by movement of plunger rod 161 relative to barrel 121. The breakable connection is strong enough to hold the proximal portion and the distal portion together during normal use of the syringe and breakable upon application of an additional force applied to the proximal portion along the longitudinal axis such as distally directed force A in Fig. 10. It is preferred that the additional distally directed force required to break the breakable connection be within the range of about 2.2 kg to 6.8 kg (5 lbs. to 15 lbs.). The proper force depends on various dimensions of the syringe barrel and plunger, the viscosity of the liquid being delivered and the mechanical and hydraulic forces encountered by the filling and delivery process. If the breakable connection is too weak the proximal and distal portions will separate during normal use of the syringe and if the force required to break the breakable connection is too high the user may not be able to easily break the breakable connection as intended. In this embodiment axial projection 169 is planar shaped having two opposed side walls with at least one transverse protuberance projecting from each of the side walls. In this embodiment, there are two transverse protuberances projecting from each side wall. This structure because of the planar axial projection and the diagonal positioning of the transverse protuberances is preferred because it creates a secure linkage between the proximal portion and the distal portion and it is strong with respect to rotational movement or rotational forces applied to the plunger and weaker with respect to axial forces applied to the plunger so that rotational forces should not break the breakable connection.

In this embodiment, proximal portion 163 is prevented from applying distally directed force to distal portion 164 after the breakable connection is broken by action of thumb press 167 on the proximal portion contacting barrel 121. In particular, a distal surface 177 on the thumb press contacts proximally directed projection 130 on the barrel to prevent unwanted contact with the distal portion.

This embodiment further includes distally directed projection 176 on the distal portion for displacing fluid in passageway 129 when the plunger is positioned distally with respect to the barrel. This is an important feature of this embodiment because it saves medication that might otherwise remain in the passageway after the injection process was completed. In an immunization program Involving millions of syringes, the amount of medication saved by the cooperative relationship between the distally directed projection and the passageway can be substantial. Even a four percent improvement can result in a free dose for every twenty-five syringes used.

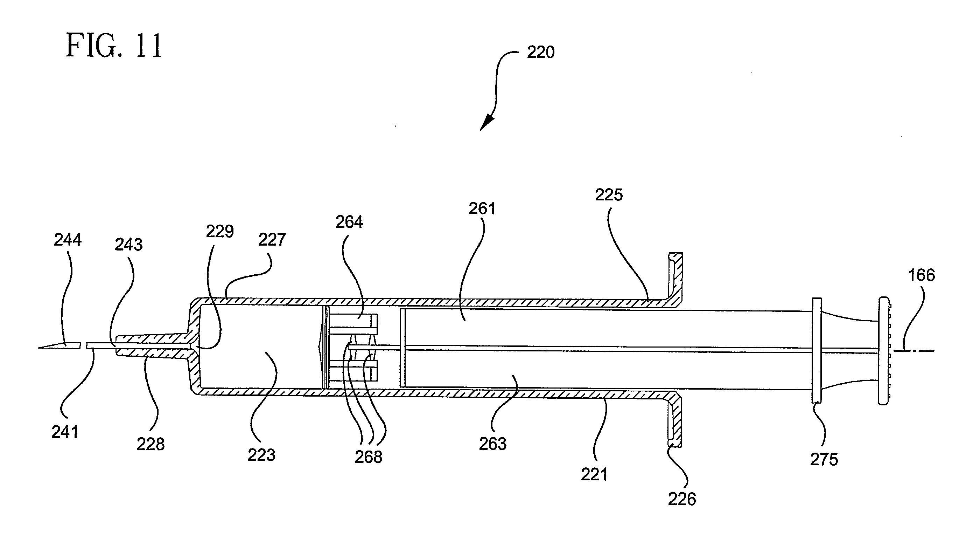

Fig. 11 illustrates another alternative embodiment of the present invention. This embodiment includes a syringe 220 which functions similarly to the syringe of the embodiment of Figs. 1-7. The syringe includes a barrel 221 having a fluid chamber 223, a proximal end 225, a distal end 227 and an elongate tip 228 extending from the distal end and having a passageway 229 therethrough in fluid communication with the chamber. The syringe further includes a cannula 241 having a proximal end 243, a distal end 244 and a lumen therethrough. The proximal end of the cannula is joined to elongate tip 228 so that the lumen is in fluid communication with passageway 229. A plunger rod 261, a proximal portion 263 and a distal portion 264 connected by a breakable connection 268.

Means for preventing proximal portion 263 from applying a distally directed force to distal portion 264 after the breakable connection is broken is provided by action between intermediate flange 275 on plunger rod 261 and barrel 221. In particular, forward motion of the proximal portion of the plunger rod is limited by contact between intermediate flange 275 and barrel flange 226 to prevent unwanted discharge of residual fluid in the chamber and/or passageway.

Cannula 241 is preferably permanently connected to the barrel so that it is not replaceable or removable during normal use. There are many ways to connect the cannula to a barrel including adhesives, such as epoxy. An advantage of this embodiment is that it can be configured to save medication from being wasted and, more importantly, when the breakable connection is broken the plunger is no longer usable and the needle cannot be removed from the syringe to be used again in an undesirable manner.

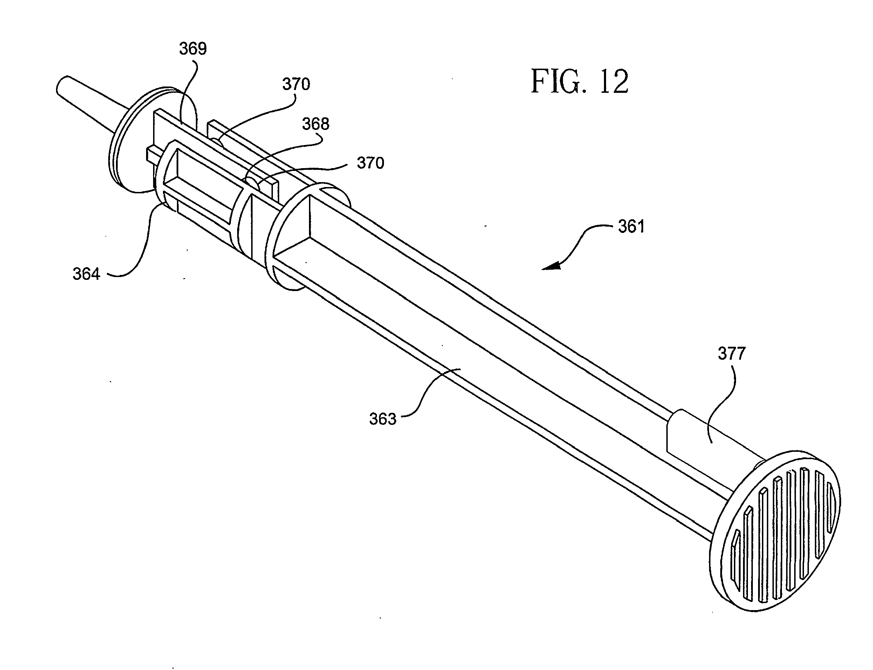

Fig. 12 illustrates an alternative elongate plunger rod 361, having a proximal portion 363 and a distal portion 364 connected by a breakable connection 368. Either the proximal portion or the distal portion includes an axial projection 369 having at least one traverse protuberance 370 projecting therefrom. In this embodiment, the axial projection is part of distal portion 364 and the protuberance is connected to the proximal portion. The breakable connection is on the protuberance and in this embodiment it is generally in the area where the transverse protuberance joins the proximal portion. Although the plunger in the embodiment of Fig. 12 functions similarly to the plunger in the embodiment of Figs. 1-7, the axial projection in this embodiment is on the distal portion while the axial projection in the embodiment of Figs. 1-7 is on the proximal portion. Accordingly, either the proximal portion or the distal portion may include the axial projection having at least one transverse protuberance. In this embodiment there are four transverse protuberances, two of which project from one side of the axial projection and the other two transverse protuberances project from the other side of the axial projection.

Means for preventing proximal portion 363 from contacting distal portion 364 after the breakable connection is broken is provided by interaction between clip on projection 377 which will contact the barrel before the proximal portion of the plunger rod can contact the distal portion of the plunger rod to prevent unwanted discharge of any residual fluid in a chamber and/or passageway. The clip-on projection can be installed after the plunger rod is formed and be made of metal or plastic or any suitable material which will adequately prevent forward motion of the plunger rod upon contact with the barrel. Projection may be made of stainless steel sheet metal having internal teeth to engage the plunger rod structure and hold it in a fixed position with respect to the proximal portion.

## Claims

1. A syringe comprising:
a barrel (21; 121) having a fluid chamber (23; 123), a proximal end (25; 125), a distal end (27; 127) and an elongate tip (28; 128) extending from said distal end having a passageway (29; 129) therethrough in fluid communication with said chamber, the barrel comprising a flange (26; 226) at its proximal end for maintaining the relative position of the barrel with respect to a plunger during fluid transfer;
a plunger rod (61; 161) having a longitudinal axis, a proximal portion (63; 163) and a distal portion (64; 164) connected by a breakable connection (68; 168), one of said proximal portion and said distal portion including an axial projection (69; 169) having at least one transverse protuberance (70; 170) projecting therefrom, said protuberance being connected to the other of said proximal portion and said distal portion, said breakable connection being on said protuberance, said distal portion including a stopper (65; 165) slidably positioned in fluid-tight engagement with an inside surface of said chamber for drawing fluid into and out of said chamber by movement of said plunger relative to said barrel, said breakable connection (68; 168) being strong enough to hold said proximal portion (63; 163) and said distal portion (64; 164) together during normal use of said syringe and breakable upon application of an additional force applied to said proximal portion along said longitudinal axis; and
a projection (73; 177) on said proximal portion (63; 163) configured to contact said barrel after said breakable connection (68) is broken, wherein said proximal projection prevents said proximal portion (63; 163) from applying a distally directed force to said distal portion (64; 164) after said breakable connection (68) is broken,
**characterized in that**
the projection (73; 177; 277; 377) has a leading edge (74) which contacts a proximal surface (30) on the flange (26;226) of the barrel and is so positioned on the proximal portion (63; 163) of the plunger rod that after breaking of the breakable connection (68; 168), the proximal portion is prevented from impacting the distal portion (64; 164), thereby avoiding squeezing out of fluid from a dead space between the plunger rod and the distal end (27; 127) of the barrel.

2. The syringe of claim 1 wherein said proximal portion (63) includes a plurality of outwardly projecting ribs (72) and said projection (73) is on at least one of said ribs.

3. The syringe of claim 2 wherein said projection (73) is integrally formed with said rib.

4. The syringe of claim 1 wherein said projection is a transverse flange (275) on said proximal portion.

5. The syringe of claim 1 wherein said axial projection (169) includes a plurality of transverse protuberances (168).

6. The syringe of claim 5 wherein said transverse protuberances (168) project from opposite sides of said axial projection (169).

7. The syringe of claim 6 wherein said axial projection (169) includes four transverse protuberances (168), two of said transverse protuberances projecting in substantially the opposite direction from the other two of said transverse protuberances.

8. The syringe of claim 1 wherein said axial projection (169) is planar shaped having two opposed side walls and at least one transverse protuberance (168) projecting from each of said two side walls.

9. The syringe of claim 1 further including a distally directed extension (176) shaped to fit within said passageway (129) for displacing fluid from said passageway when said plunger (161) is positioned distally with respect to said barrel (121).

10. The syringe of claim 1 wherein said breakable connection is made of material selected from the group of polyethylene, polystyrene, polypropylene and adhesives.

11. The syringe of claim 1 further including a needle assembly (40) including a cannula (41) having a proximal end (43), a distal end (44) and a lumen (45) therethrough, a hub (47) having an open proximal end (49) with a cavity (50) therein, and a distal end (51) joined to said proximal end of said cannula so that said lumen is in fluid communication with said cavity, said needle assembly being connected to said barrel (21) so that said elongate tip (28) of said barrel is in said cavity of said hub.

## Patentansprüche

1. Spritze mit:
einem Zylinder (21; 121) mit einer Fluidkammer (23; 123), einem proximalen Ende (25; 125), einem distalen Ende (27; 127) und einer sich von dem distalen Ende aus erstreckenden langgestreckten Spitze (28; 128) mit einem hindurchgehenden Durchlass (29; 129), der in Fluidverbindung mit der Kammer steht, wobei der Zylinder an seinem proximalen Ende einen Flansch (26; 226) zum Beibehalten der Relativposition des Zylinders in Bezug zu einem Kolben während des Fluidtransfers aufweist;
einer Kolbenstange (61; 161) mit einer Längsachse, einem proximalen Bereich (63; 163) und einem distalen Bereich (64; 164), welche durch eine zerbrechbare Verbindung (68; 168) verbunden sind, wobei einer der Bereiche, entweder der proximale Bereich oder der distale Bereich, einen axialen Vorsprung (69; 169) mit mindestens einem davon abstehenden quergerichteten Vorsprung (70; 170) aufweist, wobei der Vorsprung mit dem anderen Bereich, entweder dem proximalen Bereich oder dem distalen Bereich, verbunden ist, wobei die zerstörbare Verbindung an dem Vorsprung vorgesehen ist, wobei der distale Bereich einen Stopfen (65; 165) aufweist, der in fluiddichtem Angriff an der Innenfläche der Kammer gleitend verschiebbar ist, um durch Bewegen des Stopfens in Bezug auf den Zylinder Fluid in die und aus der Kammer zu ziehen, wobei die zerstörbare Verbindung (68; 168) ausreichend stark ist, um den proximalen Bereich (63; 163) und den distalen Bereich (64; 164) während des normalen Gebrauchs der Spritze zusammenzuhalten, und bei Aufbringen einer entlang der Längsachse gerichteten zusätzlichen Kraft auf den proximalen Bereich zerstörbar ist; und
einem Vorsprung (73; 177) an dem proximalen Bereich (63; 163), welcher derart ausgebildet ist, den Zylinder nach den Zerstören der zerstörbaren Verbindung (68) zu berühren, wobei der proximale Vorsprung den proximalen Bereich (63; 163) daran hindert, eine distal gerichtete Kraft auf den distalen Bereich (64; 164) aufzubringen, nachdem die zerstörbare Verbindung (68) zerstört ist,
**dadurch gekennzeichnet, dass**
der Vorsprung (73; 177; 277; 377) einen vorderen Rand (74) aufweist, welcher eine proximale Fläche (30) des Flanschs (26; 226) des Zylinders berührt und derart an dem proximalen Bereich (63; 163) der Kolbenstange positioniert ist, dass nach dem Zerstören der zerstörbaren Verbindung (68; 168) der proximale Bereich an einem Anstoßen gegen den distalen Bereich (64; 164) gehindert ist, wodurch das Drücken von Fluid aus einem Totraum zwischen der Kolbenstange und dem distalen Ende (27; 127) des Zylinders vermieden ist.

2. Spritze nach Anspruch 1, bei welcher der proximale Bereich (63) mehrere nach außen abstehende Rippen (72) aufweist und der Vorsprung (73) auf mindestens einer dieser Rippen vorgesehen ist.

3. Spritze nach Anspruch 2, bei welcher der Vorsprung (73) einstückig mit der Rippe ausgebildet ist.

4. Spritze nach Anspruch 1, bei welcher der Vorsprung ein quergerichteter Flansch (275) an dem proximalen Bereich ist.

5. Spritze nach Anspruch 1, bei welcher der axiale Vorsprung (169) mehrere quergerichtete Vorsprünge (168) aufweist.

6. Spritze nach Anspruch 5, bei welcher die quergerichteten Vorsprünge (168) von entgegengesetzten Seiten des axialen Vorsprungs (169) abstehen.

7. Spritze nach Anspruch 6, bei welcher der axiale Vorsprung (169) vier quergerichtete Vorsprünge (168) aufweist, wobei zwei der quergerichteten Vorsprünge in zu den anderen beiden quergerichteten Vorsprüngen im Wesentlichen entgegengesetzter Richtung abstehen.

8. Spritze nach Anspruch 1, bei welcher der axiale Vorsprung (169) planar mit zwei entgegengesetzten Seitenwänden und mindestens einem von jeder der beiden Seitenwände abstehenden quergerichteten Vorsprung (168) ausgebildet ist.

9. Spritze nach Anspruch 1, ferner mit einer distal gerichteten Verlängerung (176), die in den Durchlass (129) passend ausgebildet ist, um Fluid aus dem Durchlass zu verdrängen, wenn der Kolben (161) in Bezug auf den Zylinder (121) distal angeordnet ist.

10. Spritze nach Anspruch 1, bei welcher die zerstörbare Verbindung aus einem Material besteht, das aus der Gruppe bestehend aus Polyethylen, Polystyrol, Polypropylen und Klebern gewählt ist.

11. Spritze nach Anspruch 1, ferner mit einer Nadelanordnung (40) mit einer Kanüle (41), die ein proximales Ende (43), ein distales Ende (44) und ein hindurchgehendes Lumen (45) aufweist, einem Ansatz (47) mit einem offenen proximalen Ende (49) mit einem darin vorgesehenen Hohlraum (50), und einem distalen Ende (51), das mit dem proximalen Ende der Kanüle derart verbunden ist, dass das Lumen in Fluidverbindung mit dem Hohlraum steht, wobei die Nadelanordnung mit dem Zylinder (21) derart verbunden ist, dass die langgestreckte Spitze (28) des Zylinders sich in dem Hohlraum des Ansatzes befindet.

## Revendications

1. Seringue comprenant :
un corps (21 ; 121) ayant une chambre pour fluide (23 ; 123), une extrémité proximale (25 ; 125), une extrémité distale (27 ; 127) et une pointe allongée (28 ; 128) s'étendant de ladite extrémité distale traversée par un passage (29 ; 129) en communication fluidique avec ladite chambre, ledit corps comprenant une collerette (26 ; 226) au niveau de son extrémité proximale pour maintenir la position relative du corps par rapport à un plongeur durant le transfert de fluide ;
une tige de plongeur (61 ; 161) ayant un axe longitudinal, une partie proximale (63 ; 163) et une partie distale (64 ; 164) raccordée par un raccord cassable (68 ; 168), l'une de ladite partie proximale et de ladite partie distale comprenant une projection axiale (69 ; 169) ayant au moins une saillie transversale (70 ; 170) se projetant à partir de celle-ci, ladite saillie étant raccordée à l'autre de ladite partie proximale et de ladite partie distale, ledit raccord cassable se trouvant sur ladite saillie, ladite partie distale comprenant une butée (65 ; 165) positionnée de manière coulissante dans une relation de prise étanche aux fluides avec une surface intérieure de ladite chambre pour aspirer le fluide dans ladite chambre et l'expulser de ladite chambre par le mouvement dudit plongeur relativement au dit corps, ledit raccord cassable (68 ; 168) étant suffisamment résistant pour supporter ladite partie proximale (63 ; 163) et ladite partie distale (64 ; 164) ensemble durant l'utilisation normale de ladite seringue et cassable lors de l'application d'une force supplémentaire à ladite partie proximale le long dudit axe longitudinal ; et
une projection (73 ; 177) sur ladite partie proximale (63 ; 163) configurée pour contacter ledit corps après la rupture dudit raccord cassable (68), dans laquelle ladite projection proximale empêche ladite partie proximale (63 ; 163) d'appliquer une force dirigée distalement sur ladite partie distale (64 ; 164) après la rupture dudit raccord cassable (68),
**caractérisée en ce que**
la projection (73 ; 177 ; 277 ; 377) a un bord avant (74) qui contacte une surface proximale (30) sur la collerette (26 ; 226) du corps et est positionné sur la partie proximale (63 ; 163) de la tige de plongeur de telle sorte qu'après la rupture du raccord cassable (68 ; 168), la partie proximale ne peut pas impacter la partie distale (64 ; 164), évitant ainsi d'expulser le fluide par compression d'un espace mort entre la tige de plongeur et l'extrémité distale (27 ; 127) du corps.

2. Seringue selon la revendication 1, dans laquelle ladite partie proximale (63) comprend une pluralité de nervures se projetant vers l'extérieur (72) et ladite projection (73) se trouve au moins sur l'une desdites nervures.

3. Seringue selon la revendication 2, dans laquelle ladite projection (73) est formée en une seule pièce avec ladite nervure.

4. Seringue selon la revendication 1, dans laquelle ladite projection est une collerette transversale (275) sur ladite partie proximale.

5. Seringue selon la revendication 1, dans laquelle ladite projection axiale (169) comprend une pluralité de saillies transversales (168).

6. Seringue selon la revendication 5, dans laquelle lesdites saillies transversales (168) se projettent depuis des côtés opposés de ladite projection axiale (169).

7. Seringue selon la revendication 6, dans laquelle ladite projection axiale (169) comprend quatre saillies transversales (168), deux desdites saillies transversales se projetant dans la direction sensiblement opposée aux deux autres saillies transversales.

8. Seringue selon la revendication 1, dans laquelle ladite projection axiale (169) est de forme plane ayant deux parois latérales opposées et au moins une saillie transversale (168) se projetant à partir de chacune desdites deux parois latérales.

9. Seringue selon la revendication 1, comprenant en outre une extension dirigée distalement (176) formée pour s'adapter à l'intérieur dudit passage (129) pour déplacer le fluide dudit passage lorsque le plongeur (161) est positionné distalement relativement au dit corps (121).

10. Seringue selon la revendication 1, dans laquelle ledit raccord cassable est en un matériau sélectionné dans le groupe constitué du polyéthylène, du polystyrène, du polypropylène et des adhésifs.

11. Seringue selon la revendication 1, comprenant en outre un ensemble aiguille (40) comprenant une canule (41) ayant une extrémité proximale (43), une extrémité distale (44) et une lumière (45) à travers celle-ci, un embout (47) ayant une extrémité proximale ouverte (49) avec une cavité (50) à l'intérieur, et une extrémité distale (51) jointe à ladite extrémité proximale de ladite canule de manière à ce que ladite lumière se trouve en communication fluidique avec ladite cavité, ledit ensemble aiguille étant raccordé au dit corps (21) de telle sorte que ladite pointe allongée (28) dudit corps se trouve dans ladite cavité dudit embout.
